(19) Europäisches Patentamt
European Patent Office
Office européen des brevets

(11) Numéro de publication: **0 258 161**
**A2**

(12) # DEMANDE DE BREVET EUROPEEN

(21) Numéro de dépôt: **87420221.1**

(22) Date de dépôt: **18.08.87**

(51) Int. Cl.[4]: **C 07 D 405/06**
C 07 D 307/28,
C 07 C 43/178, A 01 N 43/50,
A 01 N 43/653

(30) Priorité: **22.08.86 FR 8612098**

(43) Date de publication de la demande:
**02.03.88 Bulletin 88/09**

(84) Etats contractants désignés:
**AT BE CH DE ES FR GB GR IT LI LU NL SE**

(71) Demandeur: **RHONE-POULENC AGROCHIMIE**
**14-20, rue Pierre Baizet**
**F-69009 Lyon (FR)**

(72) Inventeur: **Greiner, Alfred**
**31 Chemin des Aulnes**
**F-69570 Dardilly (FR)**

**Pepin, Régis**
**27, Montée Castellane**
**F-69140 Rillieux la Pape (FR)**

(74) Mandataire: **Ranguis, Patrick Frédéric et al**
**RHONE POULENC AGROCHIMIE Service DPI B.P. 9163**
**F-69263 Lyon Cédex 09 (FR)**

(54) **Dérivés de 2,5-dihydrofuranne à groupe triazole ou imidazole, procédé de préparation, utilisation comme fongicide.**

(57) Composé de formule :

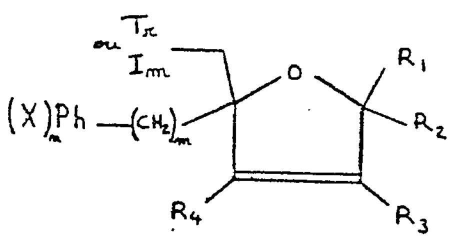

(I)

dans laquelle:
- $R_1$, $R_2$, $R_3$, $R_4$, identiques ou différents, sont l'atome d'hydrogène, ou un radical alkyle inférieur, cycloalkyle inférieur, aryle (notamment phényle), éven tuellement substitué,
- X est un atome d'halogène,
- n est un nombre entier, positif ou nul, inférieur à 6,
- m = 0 ou 1, de préférence 0,
- Tr représente un groupe 1,2,4-triazole 1-yl et Im représente un groupe 1,3-imidazolyl 1-yl
- n = 1 à 5
Utilisation comme fongicide.

EP 0 258 161 A2

**Description**

DERIVES DE 2,5-DIHYDROFURANNE A GROUPE TRAIZOLE OU IMIDAZOLE, PROCEDE DE PREPARATION, UTILISATION COMME FONGICIDE

La présente invention concerne de nouveaux composés, à usage phytosanitaire ou industriel , à groupements triazole ou imidazole et 2,5-dihydrofuranne. Elle concerne également le procédé de préparation desdits composés et les produits éventuellement utilisables à titre d'intermédiaires dans le procédé de préparation. Elle concerne ensuite l'utilisation à titre de fongicides de ces composés, les compositions fongicides à base de ces composés et les procédés pour lutter contre les maladies fongiques des cultures utilisant ces composés.

De nombreux produits à groupes triazole, notamment des fongicides, sont déjà connus en particulier par la demande de brevet européen no 151 084.

Un but de la présente invention est de proposer des composés présentant des propriétés améliorées dans le traitement des maladies fongiques, notamment des céréales.

Un autre but de la présente invention est de proposer des composés présentant un spectre d'utilisation également amélioré dans le domaine des maladies fongiques, notamment des céréales, de la vigne, des cultures légumières

Il a maintenant été trouvé que ces buts pouvaient être atteints grâce aux produits de formule I indiquée dans l'annexe se trouvant en fin de description dans laquelle:

- $R_1$, $R_2$, $R_3$, $R_4$, identiques ou différents, sont l'atome d'hydrogène, ou un radical alkyle inférieur, cycloalkyle inférieur, alcényle inférieur (notamment allyle), alcynyle inférieur (notamment propargyle), aryle (notamment phényle), les dits radicaux étant éventuellement substitués par un ou plusieurs atomes ou radicaux comme les atomes d'halogènes, les radicaux alkoxy inférieurs, aryloxy (notamment phénoxy), aryle (notamment phényle), alkyle inférieur, halogénoalkyle inférieur (notamment $CF_3$), halogénoalcoxy inférieur (notamment $OCF_3$), hydroxy,
- X est un atome d'halogène, de préférence le fluor, le brome ou le chlore, ou un groupe alkyle ou alkoxy ayant de 1 à 12 atomes de carbone, de préférence de 1 à 4 atomes de carbone, et étant éventuellement mono ou polyhalogéné (groupement $CF_3$ notamment), ou un groupe cyano,
- n est un nombre entier, positif ou nul, inférieur à 6, étant entendu que lorsque n est supérieur à 1, les substituants X peuvent être soit identiques, soit différents,
-Ph est le noyau phényle, éventuellement substitué (n= 1 à 5),
- m = 0 ou 1, de préférence 0,
- Tr représente un group 1,2,4-triazole 1-yl et Im représente un groupe 1,3-imidazolyl 1-yl

L'invention concerne également les formes salifiées des composés selon l'invention. Les formes salifiées sont les formes acceptables en agriculture parmi lesquelles on peut citer : les chlorhydrate, sulfate, oxalate, nitrate, aryl ou alkylsulfonate ainsi que les complexes d'addition de ces composés avec des sels métalliques, notamment des sels de fer, chrome, cuivre, manganèse, zinc, cobalt, étain, magnésium et aluminium.

A titre d'exemple, des complexes avec le zinc peuvent être obtenus en faisant réagir le composé de formule I avec le chlorure de zinc.

Au sens du présent texte, on entend que l'adjectif inférieur, lorsqu'il qualifie un radical organique, signifie que ce radical comporte au plus six atomes de carbone. Ce radical peut être linéaire ou ramifié.

Le déposant souhaite souligner que la planche en fin de description ne saurait en aucune manière être considérée comme un dessin mais comme faisant partie intégrante de la description de l'invention.

Les composés de formule I et les composés éventuellement utilisables à titre d'intermédiaires dans les procédés de préparation et qui seront définis à l'occasion de la description de ces procédés peuvent exister sous une ou plusieurs formes d'isomères selon le nombre de centres asymétriques de la molécule. L'invention concerne donc aussi bien tous les isomères optiques que leurs mélanges racémiques et les diastéréoisomères correspondants. La séparation des diastéréoisomères et/ou des isomères optiques peut s'effectuer selon les méthodes connues en soi.

En vue des applications fongicides il a été trouvé que l'invention concernait de préférence les composés de formule I dans laquelle X est un halogène et n = 1, 2 ou 3.

Il a été également trouvé qu'il était préférable d'utiliser les composés de formule I dans laquelle n = 1 ou 2, et X est un atome d'halogène placé en ortho et/ou en para.

De préférence encore n = 2 et X est un atome d'halo gène, avantageusement le chlore placé en ortho et en para.

De préférence encore $R_1$, $R_2$ correspondent à l'atome d'hydrogène et avantageusement :

$R_3$, $R_4$ identiques ou différents correspondent à un radical alkyle inférieur,

ou

$R_3$ correspond à un radical alkyle inférieur et $R_4$ est l'atome d'hydrogène

ou

$R_4$ correspond à un radical alkyle inférieur, et $R_3$ est l'atome d'hydrogène.

De préférence encore les composés comportent un groupe 1,2,4-triazole 1-yl.

La présente invention concerne également des procédés de préparation de ces composés. Un procédé est caractérisé en ce qu'il consiste dans les étapes suivantes :

- hydrogénation, dans le cas où $R_3$ et $R_4$ correspondent à l'atome d'hydrogène, d'un composé de formule IV, dans laquelle X, n, m, $R_1$, $R_2$ ont la même signification que dans le composé de formule I, Hal correspond à un atome d'halogène, Pr représente un groupe protecteur, comme le 1-éthoxy éthyle, ou l'atome d'hydrogène, ou dans le cas où $R_3$ et/ou $R_4$ sont différents de l'atome d'hydrogène, addition d'un organomagnésien de formule $R_4MgX$ sur le composé de formule IV puis éventuellement addition d'un halogénure d'alkyle de formule $R_3X$,
- cyclisation du composé de formule III dans laquelle $R_1$, $R_2$, $R_3$, $R_4$, X, n, m ont la même signification que dans le composé de formule I, Hal a la même signification que dans le composé de formule IV, Z est un atome d'hydrogène ou OZ est un groupe partant
- greffage d'un noyau imidazole ou triazole sur le composé de formule II dans laquelle $R_1$, $R_2$, $R_3$, $R_4$, X, m, n ont la même signification que précédemment et Y correspond à un atome ou groupement susceptible d'être éliminé au moyen d' une substitution nucléophile, éventuellement aprés transformation intermédiaire appropriéé.

Dans le cas où les groupements $R_3$ et $R_4$ correspondent tous les deux à l'atome d'hydrogène, de préférence les composés de formule III sont obtenus par hydrogénation du composé de formule IV au moyen d'une quantité équimoléculaire d'hydrogène en présence d'un catalyseur approprié éventuellement empoisonné choisi parmi les catalyseurs suivants : palladium, ruthénium, nickel de Raney, platine, rhodium et de préférence le palladium éventuellement empoisonné (pyridine, quinoléine) qui donne spécifiquement l'oléfine cis.

Cette réaction peut être effectuée en phase homogène ou hétérogène.

De préférence on choisira le palladium à l'état métallique déposé sur un support inerte tel que le noir de carbone, le carbonate de calcium ou le sulfate de baryum.

La réaction bien que cela ne soit pas indispensable peut avantageusement être effectuée en solvant polaire protique par exemple dans un alcool inférieur tel que le méthanol ou aprotique par exemple dans le toluène.

La dilution du composé de formule IV est de préférence comprise entre 1 et 80 % en poids ou mieux 5 % -40 % par rapport à la solution totale.

De même si la proportion molaire de catalyseur par rapport au composé de formule IV peut varier de manière considérable il sera préférable pour des raisons industrielles évidentes d'utiliser le catalyseur dans une proportion molaire comprise entre 0,01 et 0,5 % par rapport au composé de formule IV.

La réaction s'effectue à des températures comprises entre -20° C et +150° C de préférence entre 15 et 80° C et sous pression de 1 à 10 atmosphères (soit 0,1 à 1 MPa).

dans le cas où Pr est un groupe protecteur, il est nécessaire de déprotéger l'alcool avant la transformation en groupe OZ et la cyclisation.

Le composé de formule IV peut être obtenu par des procédés bien connus de l'homme de l'art par exemple par addition de l'organométallique de formule :

$R_2$-(OPr)CH-C≡C-M

dans laquelle M est un métal alcalin ou un magnésien (Mg Hal) ou un zincique (Zn Hal) par exemple sur une acétophénone de formule :

$(X)n\ Ph(CH_2)m\ COCH_2Hal$

La réaction peut s'effectuer par exemple dans le tétrahydrofuranne de manière connue.

Dans le cas ou au moins un des groupes $R_3$ ou $R_4$ ne correspondent pas à l'atome d'hydrogène, il est possible d'obtenir les composés de formule III par la voie de synthèse suivante : addition d'un organomagnésien de formule $R_4MgX$ (de préférence 2 à 10 moles pour 1 mole de IV) sur les composés de formule générale IV, en présence ou non d'iodure de cuivre (de préférence entre -20° et + 80° C et 0,01 à 5% en mole) suivie éventuellement de l'addition d'un halogénure d'alkyle $R_3X$ (1 à 10 moles pour 1 mole de composé sur lequel a été fixé $R_4MgX$ ) dans un solvant tel que le tétrahydrofuranne de préférence entre - 20° et + 80° C). Dans le cas où l'on s'arrête à l'addition de $R_4MgX$ il est bien évident que l'on fait suivre cette étape d'addition d'une hydrolyse.

Un autre procédé d'obtention des composés de formule III dans lesquels le radical Z est l'atome d'hydrogène consiste à faire réagir un organomagnésien de formule $R_3$Mg Hal sur un alcool acétylénique de formule $R_4$-C = C-$CR_1R_2$-OH dans lesquelles $R_3$, $R_4$ ont la même signification que précédemment dans un solvant choisi parmi les éthers, en particulier le tétrahydrofuranne, ou les hydrocarbures comme le benzène ou le toluène en présence d'un halogénure cuivreux éventuellement complexé par un dialkylsulfure et en quantité catalytique (de 0,1 à 20 % en mole par rapport à l'organomagnésien $R_3$ Mg Hal). Cet organomagnésien lui-même est utilisé en quantité molaire double par rapport à l'alcool acétylènique engagé, à des températures comprises entre - 60° et + 50°, de préférence entre - 30° et + 20°. On prépare ainsi un réactif organomagnésien de formule XIV selon les indications contenues dans la littérature : J.F. Normant, A. Alexakis Synthesis (1981) 841-870.

Cet organomagnésien de formule XIV est condensé avec une halogénoacétophénone de formule $(X)_n$ Ph $(CH_2)_m$ $COCH_2Hal$, Ph étant un noyau phényle, dans des conditions similaires aux procédures précédentes permettant d'obtenir les composés de formule III.

Un composé de formule III peut aussi être converti en composé de formule I par la succession de réactions suivantes :

- le composé de formule III (Z = H) est acylé selon des conditions bien connues de l'homme de l'art (par exemple par un halogénure d'acyle aliphatique ou aromatique ou encore un anhydride en présence d'une base telle que la pyridine) pour conduire au composé III (Z = acyle),

- ce dernier est ensuite condensé avec un azole pour obtenir un composé XIII à partir d'un composé III dans les conditions qui seront décrites ci-aprés,
- le groupe Z est ensuite saponifié dans les conditions habituelles connues de l'homme de l'art,
- la cyclisation de XIII peut s'effectuer alors selon les procédures indiquées ci-aprés,
- dans des cas particuliers (notamment dans les cas où $(X)_n$ constitue une substitution en ortho du noyau aromatique et si m = o) les composés I sont formés directement à partir des composés III (Z = acyle) dans les conditions de condensation de l'azole et les composés XIII (Z = acyle) ne sont alors pas isolés.

Un procédé avantageux mais non obligatoire pour synthétiser les composés de formule II dans le cas où Y correspond à un atome d'halogène consiste à cycliser le composé de formule III sous forme cis dans laquelle X, n, m, $R_2$, $R_3$, $R_4$ ont la même signification que pour le composé de formule I, soit en milieu acide si Z est un atome d'hydrogène, soit en milieu basique si Z est un groupe partant tel que un mésylate, un tosylate, un triflate, un groupement de formule $[Ph_3P^+ - O^-]$.

Ces groupes partants sont fixés au groupe hydroxyle de manière connue et de façon sélective sans toucher au groupe hydroxyle tertiaire.

Les catalyseurs acides organiques ou minéraux conviennent pour cette cyclisation. Ceux-ci peuvent être solubles dans le milieu réactionnel ou insolubles, protiques ou aprotiques. Comme acides protiques on peut citer les acides chlorhydrique, sulfurique, trifluoroacétique, perchlorique, benzène-sulfonique, toluène-sulfonique, méthane-sulfonique. Comme acides aprotiques on peut citer les acides de Lewis tels que $BF_3$, $AlCl_3$ et $SnCl_4$.

On utilisera de préférence de 0,1 à 2 équivalent molaire d'acides.

On peut également effectuer cette cyclisation au moyen de catalyseurs fixés sur des supports inertes tels que les résines sulfoniques.

La cyclisation s'effectue ordinairement par simple chauffage des réactifs indiqués. La température est généralement comprise entre 10° C et 100° C ou si il y a un solvant entre 10° C et la température d'ébullition du solvant considéré.

Parmi les nombreux solvants utilisables on peut citer les solvants aliphatiques, aromatiques tel que le toluène, les éthers et les cétones.

Dans le cas de la cyclisation en milieu basique, on peut mentionner à titre indicatif des bases minérales comme par exemple la soude ou la potasse, les carbonates de métaux alcalins ou de métaux alcalinoterreux, des bases azotées comme la triéthylamine, des amines quaternaires comme le tétrabutylammonium hydroxyde, ou des hydroxydes de phosphonium. On utilisera de préférence de 0,1 à 2 équivalent molaire de base. On peut également effectuer cette cyclisation au moyen de catalyseurs fixes sur des supports inertes tels que les résines. La réaction s'effectue ordinairement à une température comprise entre 10° C et 100° C et éventuellement en présence d'un solvant tel que les solvants aliphatiques, aromatiques, les éthers, les cétones.

Les composés de formule II dans laquelle Y correspond à un groupement hydroxy peuvent être obtenus par traitement basique des produits de formule générale III dans laquelle Z = H.

On passe alors par l'intermédiaire des époxydes VII correspondants issus des produits de formule générale générale VI. Il est par la suite nécessaire après l'isomérisation de transformer le groupe Y = hydroxy en groupe partant (mesylate, tosylate, triflate, $PH_3P^+$-0) avant la réaction de subtitution par le triazole comme cela sera décrit ci-après.

L'étape de greffage est avantageusement effectuée en présence d'un accepteur d'acide, en milieu anhydre ou non anhydre, dans un solvant, inerte dans les conditions de la réaction, généralement entre 50 et 180° C et de préférence à une température voisine de la température d'ébullition du solvant. Comme accepteurs d'acide, on peut mentionner des bases minérales comme par exemple la soude ou la potasse, les carbonates de métaux alcalins ou de métaux alcalinoterreux, des bases azotées comme la triéthylamine, des amines quaternaires comme le tétrabutylammonium hydroxyde, ou des hydroxydes de phosphonium. Comme solvants on utilise avantageusement des solvants aprotiques polaires, tels que par exemple, le diméthylformamide, le diméthylacétamide, le diméthylsulfoxyde, l'acétone, la méthyléthyl-cétone, la méthyl-isobutylcétone, l'acétonitrile, la N-méthylpyrrolidone ou des solvants protiques polaires tels que le méthanol ou le propanol ou le butanol. Si désiré, cette réaction peut être effectuée en présence d'un catalyseur approprié. Comme catalyseur utilisable, on peut mentionner des catalyseurs de transfert de phases, tels que par exemple, des dérivés d'ammonium quaternaires comme les halogénures de tétrabutylammonium et de préférence les iodures.

La réaction est de préférence effectuée en excès molaire de dérivé de triazole ou imidazole de préférence compris entre 1,05 et 1,5.

Lorsque l'on utilise un solvant, on préfèrera travailler dans un milieu dilué comportant 1 % à 70 % en poids de composé de formule II par rapport à la solution totale.

L'accepteur d'acide est au moins présent en quantité stoechiométrique en équivalent d'atome d'hydrogène labile du triazole ou imidazole. En général un rapport en équivalent molaire compris entre 1 et 2,5 sera satisfaisant.

Il est évident que le dérivé salifié du triazole ou imidazole peut être préparé à part et dans ce cas la présence d'un accepteur d'acide n'est pas requise pour la réaction de greffage. Elle s'effectue en milieu anhydre ou non anhydre dans un solvant dans les mêmes conditions opératoires que celles décrites dans le cas de la formation in situ du dérivé triazole ou imidazole salifié.

Un autre procédé de préparation consiste à faire réagir une halogénoacetophénone de formule : $(X)_n$Ph $(CH_2)_m$ CO $CH_2$Hal avec un organométallique de formule $R_2R_1C = CR_3$- $CHR_4M$ dans lesquelles X, m, n, Hal, $R_1$, $R_2$, $R_3$, $R_4$ ont la même signification que dans les formules I et III ci-dessus et M correspond à un métal alcalin ou à un magnésien (Mg Hal) ou à un zincique (Zn Hal) par exemple, dans un solvant, choisi de préférence parmi les éthers, tels que l'éther diéthylique ou le tétrahydrofuranne, les hydrocarbures aliphatiques, alicycliques ou aromatiques tels que l'hexane, le toluène à une température choisie entre -50° C et le reflux du solvant considéré et dans un rapport molaire cétone : organométallique compris de préférence entre 1,1 et 0,2. La réaction conduit au composé de formule V après neutralisation du milieu réactionnel.

On fait réagir ensuite le composé de formule V avec un imidazole ou un triazole non substitué en présence de base organique ou minérale par exemple la pyridine, la triéthylamine, la soude, la potasse, les carbonates et bicarbonates de métaux alcalins ou alcalino terreux et dans un solvant approprié tel que par exemple les alcools, les cétones, les amides, les nitriles, les hydrocarbures aromatiques éventuellement halogénés, à une température comprise entre 80° et le reflux du solvant et dans un rapport molaire V : imidazole ou triazole de préférence compris entre 1,1 et 0,2, ce qui conduit au composé de formule VI. La réaction passe en général par un intermédiaire époxyde de formule VII qui peut éventuellement être isolé ou préparé séparémment par des méthodes connues de l'homme de l'art.

On additionne sur le composé VI de préférence mole à mole une molécule d'halogène ou d'halogène mixte dans un solvant inerte tel que les hydrocarbures saturés ou aromatiques éventuellement halogénés ce qui conduit au composé VIII. Le composé IX est obtenu de préférence à température ambiante par cyclisation du composé VIII en présence d'une base organique ou minérale indiquée précédemment dans un rapport molaire composé VIII : base de préférence compris entre 1,1 et 0,66. La réaction peut s'effectuer en milieu solvant protique ou aprotique (eau, alcool, cétone, nitrile, ester, hydrocarbure saturé ou aromatique éventuellement halogéné, diméthylsulfoxyde, amide comme le diméthylformamide).

Un autre procédé de préparation des composés IX consiste à placer l'étape de greffage du noyau imidazole ou triazole après l'étape de cyclisation en utilisant le même mode opératoire pour les différentes étapes. Ainsi on additionne sur le composé V une molécule d'halogène ou d'halogènure d'halogène (halogène mixte) pour donner le composé X, celui-ci étant ensuite cyclisé pour donner le composé XI, qui est ensuite pourvu d'un groupement triazole ou imidazole pour donner le composé IX.

Pour obtenir le composé I on fait réagir le composé IX avec une base. On peut mentionner des bases minérales comme par exemple la soude ou la potasse, les carbonates de métaux alcalins ou de métaux alcalinoterreux, des bases azotées comme la triéthylamine, des amines quaternaires comme le tétrabutylammonium hydroxyde, ou des hydroxydes de phosphonium. On utilisera de préférence 1 à 5 équivalent molaire de base par mole de composé IX. La réaction s'éffectue avantageusement en présence d'un solvant. Comme solvants on utilise avantageusement des solvants aprotiques polaires, tels que par exemple, le diméthylformamide, le diméthylacétamide, la diméthylsulfoxyde, l'acétone, la méthyléthyl-cétone, la méthyl-isobutylcétone, l'acétonitrile, la N-méthylpyrrolidone ou des solvants protiques polaires tels que le méthanol ou la propanol ou le butanol. La température est généralement comprise entre la température ambiante et le reflux du solvant.

Un autre procédé consiste à greffer le noyau triazole ou imidazole sur le composé IV ou III de manière à obtenir les composés de formule XII et XIII respectivement puis à hydrogéner dans le cas du composé XII et à cycliser dans les mêmes conditions que celles indiquées précédemment

Le composé de formule XIII (Z = H), peut également être obtenu par réaction d'une azolyl (Tr ou Im) acétophéno ne, obtenue par greffage d'un noyau triazole ou imidazole sur l'halogénoacétophénone de formule $(X)_n$ Ph $(CH_2)_m$ $COCH_2$ Hal dans les conditions précitées, avec un organomagnésien de formule XIV.

Un autre procédé consiste à traiter le composé de formule X par un imidazole ou triazole en présence d'une base en excés (2 moles ou plus), ce qui conduit directement au composé IX.

On peut de même halogéner le composé VII et greffer l'azole ensuite pour obtenir le composé VIII.

En fin de réaction, quelque soit le procédé utilisé, le composé formé est isolé du milieu réactionnel par tout moyen en soi connu tel que, par exemple par distillation du solvant, ou par cristallisation du produitdans le milieu réactionnel, ou par filtration, puis si nécessaire, ce composé est purifié par des méthodes usuelles comme recristallisation dans un solvant approprié.

La présente invention concerne les composés de formule II à XIII, dans lesquelles X, m, n, $R_1$ à $R_4$, Hal, W, Z, Pr, Y ont la même signification que précédemment.

La présente invention concerne aussi l'utilisation des composés de formule I à titre de fongicides.

Les composés selon l'invention peuvent être utilisés pour la lutte tant préventive que curative contre les champignons, notamment de type basidiomycètes, ascomycètes, adelomycètes ou fungi-imperfecti, en particulier les rouilles, oïdium, piétin-verse, fusarioses, helminthosporioses, septorioses, rhizoctones des végétaux et des plantes en général et en particulier des céréales telles que le blé, l'orge, le seigle, l'avoine et leurs hybrides et aussi le riz et le maïs. Les composés selon l'invention sont actifs en particulier contre les champignons notamment de type basidiomycètes, ascomycètes, adelomycètes ou fungi-imperfecti comme Botrytis cinerea, Erysiphe graminis, Puccinia recondita, Piricularia oryzae, Cercospora beticola, Puccinia striiformis, Erysiphe cichoracearum, Fusarium oxysporum (melonis), Pyrenophora avenae, Septoria tritici, Venturia inaequalis, Whetzelinia sclerotiorum, Monilia laxa, Mycosphaerella fijiensis, Marssonina panettoniana, Alternaria solani, Aspergillus niger, Cercospora arachidicola, Cladosporium herbarum, Helminthosporium oryzae, Penicillium expansum, Pestalozzia sp, Phialophora cinerescens, Phoma betae, Phoma foveata, Phoma

lingam, Ustilago maydis, Verticillium dahliae, Ascochyta pisi, Guignardia bidwellii, Corticium rolfsii, Phomopsis viticola, Sclerotinia sclerotiorum, Sclerotinia minor, Coryneum cardinale, Rhizoctonia solani.

Ils sont aussi et encore actifs contre les champignons suivants : Acrostalagmus koningi, les Alternaria, les Colletotrichum, Corticium rolfsii, Diplodia natalensis, Gaeumannomyces graminis, Gibberella fujikuroi, Hormodendron cladosporioides, Lentinus degener ou tigrinus, Lenzites quercina, Memnoniella echinata, Myrothecium verrucaria, Paecylomyces varioti, Pellicularia sasakii, Phellinus megaloporus, Polystictus sanguineus, Poria vaporaria, Sclerotium rolfsii, Stachybotris atra, les Stereum, Stilbum sp. Trametes trabea, Trichoderma pseudokoningi, Trichothecium roseum.

Les composés de l'invention sont spécialement intéressants par leur spectre large au niveau des maladies des céréales (oïdium, rouille, piétin-verse, helminthosporioses, septorioses et fusarioses). Ils présentent également un grand intérêt en raison de leur activité sur la pourriture grise (Botrytis) et les cercosporioses, et, de ce fait, ils peuvent être appliqués sur des cultures aussi variées que la vigne, les cultures maraîchères et l'arboriculture et les cultures tropicales telles que l'arachide, le bananier, le caféier, la noix de pécan et d'autres.

Outre les applications déjà décrites plus haut, les produits selon l'invention présentent en outre une excellente activité biocide à l'égard de nombreuses autres variétés de microorganismes parmi lesquelles on peut citer à titre non limitatif, des champignons comme ceux des genres :
- Pullularia comme l'espèce P. pullulans,
- Chaetomium comme l'espèce C. globosum,
- Aspergillus comme l'espèce Aspergillus niger,
- Coniophora comme l'espèce C. puteana.

En raison de leur activité biocide, les produits de l'invention permettent de combattre efficacement les microorganismes dont la prolifération crée de nombreux problèmes dans les domaines agricole et industriel. A cet effet, ils conviennent tout spécialement bien à la protection des végétaux ou de produits industriels tels que le bois, le cuir, les peintures, le papier, les cordages, les plastiques, les circuits d'eau industriels.

Ils sont tout particulièrement bien adaptés à la protection des produits lignocellulosiques et notamment du bois, qu'il s'agisse de bois d'ameublement, de charpente ou de bois exposé aux intempéries tels que les bois de clôture, les piquets de vignes, les traverses de chemin de fer.

Les composés selon l'invention utilisés seuls ou sous la forme de compositions telles que définies ci-dessus dans les traitements du bois, sont généralement mis en oeuvre avec des solvants organiques et peuvent être éventuellement associés à un ou plusieurs produits biocides connus tels que le pentachlorophénol, les sels métalliques, notamment de cuivre, de manganèse, de cobalt, de chrome, de zinc dérivés d'acides minéraux ou carboxyliques (acides heptanoïque, octanoïque, naphténique); les complexes organiques de l'étain, le mercaptobenzothiazole, les insecticides tels que les pyrethroïdes ou les organochlorés.

Ils présentent enfin une excellente sélectivité vis-à-vis des cultures.

Ils s'appliquent avantageusement à des doses de 0,005 à 5 kg/ha, et plus spécifiquement de 0,01 à 0,5 kg/ha.

Pour leur emploi pratique, les composés selon l'invention sont rarement utilisés seuls. Le plus souvent ils font partie de compositions. Ces compositions, utilisables pour la protection des végétaux contre les maladies fongiques, ou dans les compositions régulatrices de la croissance des plantes, contiennent comme matière active un composé selon l'invention tel que décrit précédemment en association avec les supports solides ou liquides, acceptables en agriculture et/ou les agents tensio-actifs également acceptables en agriculture. En particulier sont utilisables les supports inertes et usuels et les agents tensio-actifs usuels.

Par le terme "support", dans le présent exposé, on désigne une matière organique ou minérale, naturelle ou synthétique, avec laquelle la matière active est associée pour faciliter son application sur la plante, sur des graines ou sur le sol. Ce support est donc généralement inerte et il doit être acceptable en agriculture, notamment sur la plante traitée. Le support peut être solide (argiles, silicates naturels ou synthétiques, silice, résines, cires, engrais solides, etc...) ou liquide (eau, alcools, cétones, fractions de pétrole, hydrocarbures aromatiques ou paraffiniques, hydrocarbures chlorés, gaz liquéfiés, etc...).

L'agent tensioactif peut être un agent émulsionnant, dispersant ou mouillant de type ionique ou non ionique. On peut citer par exemple des sels d'acides polyacryliques, des sels d'acides lignosulfoniques, des sels d'acides phénolsulfoniques ou naphtalènesulfoniques, des polycondensats d'oxyde d'éthylène sur des alcools gras ou sur des acides gras ou sur des amines grasses, des phénols substitués (notamment des alkylphénols ou des arylphénols), des sels d'esters d'acides sulfosucciniques, des dérivés de la taurine (notamment des alkyltaurates), des esters phosphoriques d'alcools ou de phénols polyoxyéthylés. La présence d'au moins un agent tensioactif est généralement indispensable lorsque la matière active et/ou le support inerte ne sont pas solubles dans l'eau et que l'agent vecteur de l'application est l'eau.

Pour leur application, les composés de formule (I) se trouvent donc généralement sous forme de compositions ; ces compositions selon l'invention sont elles-mêmes sous des formes assez diverses, solides ou liquides.

Comme formes de compositions solides, on peut citer les poudres pour poudrage ou dispersion (à teneur en composé de formule (I) pouvant aller jusqu'à 100 %) et les granulés, notamment ceux obtenus par extrusion, par compactage, par imprégnation d'un support granulé, par granulation à partir d'une poudre (la teneur en composé de formule (I) dans ces granulés étant entre 1 et 80 % pour ces derniers cas).

Comme formes de compositions liquides ou destinées à constituer des compositions liquides lors de l'application, on peut citer les solutions, en particulier les concentrés solubles dans l'eau, les concentrés émulsionnables, les émulsions, les suspensions concentrées, les aérosols, les poudres mouillables (ou poudre à pulvériser), les pâtes.

Les concentrés émulsionnables ou solubles comprennent le plus souvent 10 à 80 % de matière active, les émulsions ou solutions prêtes à l'application contenant, quant à elles, 0,01 à 20 % de matière active.

Ces compositions peuvent contenir aussi toute sorte d'autres ingrédients tels que, par exemple, des colloïdes protecteurs, des adhésifs, des épaississants, des agents thixotropes, des agents de pénétration, des stabilisants, des séquestrants, etc... ainsi que d'autres matières actives connues à propriétés pesticides (notamment insecticides ou fongicides) ou à propriétés favorisant la croissance des plantes (notamment des engrais) ou à propriétés régulatrices de la croissance des plantes. Plus généralement les composés selon l'invention peuvent être associés à tous les additifs solides ou liquides correspondant aux techniques habituelles de la mise en formulation.

Par exemple, en plus du solvant, les concentrés émulsionnables peuvent contenir quand c'est nécessaire, 2 à 20 % d'additifs appropriés comme les stabilisants, les agents tensio-actifs, les agents de pénétration, les inhibiteurs de corrosion, les colorants ou les adhésifs précédemment cités.

Les doses d'emploi dans le cas d'une utilisation comme fongicides des composés selon l'invention peuvent varier dans de larges limites, notamment selon la virulence des champignons et les conditions climatiques.

D'une manière générale des compositions contenant 0,5 à 5000 ppm de substance active conviennent bien ; ces valeurs sont indiquées pour les compositions prêtes à l'application. Ppm signifie "partie par million". La zone de 0,5 à 5000 ppm correspond à une zone de $5x10^{-5}$ à 0,5 % (pourcentages pondéraux).

En ce qui concerne les compositions adaptées au stockage et au transport, elles contiennent plus avantageusement de 0,5 à 95 % (en poids) de substance active.

Ainsi donc, les compositions à usage agricole selon l'invention peuvent contenir les matières actives selon l'invention dans de très larges limites, allant de $5.0^{-5}$% à 95 % (en poids).

A titre d'exemple, voici la composition de quelques concentrés :

<u>Exemple F (formulation) 1</u>

- matière active 400 g/l
- dodécylbenzène sulfonate alcalin 24 g/l
- nonylphénol oxyéthylé à 10 molécules d'oxyde d'éthylène 16 g/l
-cyclohexanone 200 g/l
- solvant aromatique q.s.p 1 litre

Selon une autre formule de concentré émulsionnable, on utilise :

<u>Exemple F 2</u> :

- matière active 250 g
- huile végétale époxydée 25 g
- mélange de sulfonate d'alcoylaryle et d'éther de polyglycol et d'alcools gras 100 g
- diméthylformamide 50 g
- xylène 575 g

A partir de ces concentrés, on peut obtenir par dilution avec de l'eau des émulsions de toute concentration désirée, qui conviennent particulièrement à l'application sur les feuilles.

Les suspensions concentrées, également applicables en pulvérisation, sont préparées de manière à obtenir un produit fluide stable ne se déposant pas et elles contiennent habituellement de 10 à 75 % de matière active, de 0,5 à 15 % d'agents tensioactifs, de 0,1 à 10 % d'agents thixotropes, de 0 à 10 % d'additifs appropriés, comme des anti-mousses, des inhibiteurs de corrosion, des stabilisants, des agents de pénétration et des adhésifs et, comme support, de l'eau ou un liquide organique dans lequel la matière active est peu ou pas soluble : certaines matières solides organiques ou des sels minéraux peuvent être dissous dans le support pour aider à empêcher la sédimentation ou comme antigels pour l'eau.

Les poudres mouillables (ou poudre à pulvériser) sont habituellement préparées de manière qu'elles contiennent 20 à 95 % de matière active, et elles contiennent habituellement, en plus du support solide, de 0 à 5% d'un agent mouillant, de 3 à 10 % d'un agent dispersant, et, quand c'est nécessaire, de 0 à 10 % d'un ou plusieurs stabilisants et/ou autres additifs, comme des agents de pénétration, des adhésifs, ou des agents antimottants, colorants, etc...

A titre d'exemple, voici diverses compositions de poudres mouillables :

<u>Exemple F 3</u> :

- matière active 50 %
- lignosulfonate de calcium (défloculant) 5 %
- isopropylnaphtalène sulfonate (agent mouillant anionique) 1 %
- silice antimottante 5 %
- kaolin (charge) 39 %

Une autre composition de poudre à pulvériser à 70 % utilise les constituants suivants :

<u>Example F 4</u> :

- matière active 700 g
- dibutylnaphtylsulfonate de sodium 50 g
- produit de condensation en proportions 3/2/1 d'acide naphtalène sulfonique, d'acide phénolsulfonique et de

formaldéhyde 30 g
- kaolin 100 g
- craie de champagne 120 g

Une autre composition de poudre à pulvériser à 40 % utilise les constituants suivants :

Exemple F 5 :
- matière active 400 g
- lignosulfonate de sodium 50 g
- dibutylnaphtalène sulfonate de sodium 10 g
- silice 540 g

Une autre composition de poudre à pulvériser à 25 % utilise les constituants suivants :

Exemple F 6 :
- matière active 250 g
- lignosulfonate de calcium 45 g
- mélange équipondéral de craie de Champagne et d'hydroxyéthylcellulose 19 g
- dibutylnaphtalène sulfonate de sodium 15 g
- silice 195 g
- craie de Champagne 195 g
- kaolin 281 g

Une autre composition de poudre à pulvériser à 25 % utilise les constituants suivants :

Exemple F 7 :
- matière active 250 g
- isooctylphénoxy-polyoxyéthylène-éthanol 25 g
- mélange équipondéral de craie de Champagne et d'hydroxyéthylcellulose 17 g
- aluminosilicate de sodium 543 g
- kieselguhr 165 g

Une autre composition de poudre à pulvériser à 10 % utilise les constituants suivants :

Exemple F 8 :
- matière active 100 g
-mélange de sels de sodium de sulfates d'acides gras saturés 30 g
- produit de condensation d'acide naphtalène sulfonique et de formaldéhyde 50 g
- kaolin 820 g

Pour obtenir ces poudres à pulvériser ou poudres mouillables, on mélange intimement les matières actives dans des mélangeurs appropriés avec les substances additionnelles et on broie avec des moulins ou autres broyeurs appropriés. On obtient par là des poudres à pulvériser dont la mouillabilité et la mise en suspension sont avantageuses ; on peut les mettre en suspension avec de l'eau à toute concentration désirée et ces suspensions sont utilisables très avantageusement en particulier pour l'application sur les feuilles des végétaux.

A la place des poudres mouillables, on peut réaliser des pâtes. Les conditions et modalités de réalisation et d'utilisation de ces pâtes sont semblables à celles des poudres mouillables ou poudres à pulvériser.

Comme cela a déjà été dit, les dispersions et émulsions aqueuses, par exemple les compositions obtenues en diluant à l'aide d'eau une poudre mouillable ou un concentré émulsionnable selon l'invention, sont comprises dans le cadre général de la présente invention. Les émulsions peuvent être du type eau-dans-l'huile ou huile-dans-l'eau et elles peuvent avoir une consistance épaisse comme celle d'une "mayonnaise".

Les granulés destinés à être disposés sur le sol sont habituellement préparés de manière qu'ils aient des dimensions comprises entre 0,1 et 2 mm et ils peuvent être fabriqués par agglomération ou imprégnation. En général, les granulés contiennent 0,5 à 25 % de matière active et 0 à 10 % d'additifs comme des stabilisants, des agents de modification à libération lente, des liants et des solvents.

Selon un exemple de composition de granulé, on utilise les constituants suivants :

Exemple F 9 :
- matière active 50 g
- épichlorhydrine 2,5 g
- éther de cétyle et de polyglycol 2,5 g
- polyéthylène glycol 35 g
- kaolin (granulométrie : 0,3 à 0,8 mm) 910 g.

Dans ce cas particulier on mélange la matière active avec l'épichlorhydrine et on dissout avec 60 g d'acétone ; on ajoute alors le polyéthylène glycol et l'éther de cétyle et de polyglycol. On arrose le kaolin avec la solution obtenue et on évapore ensuite l'acétone sous vide. On utilise avantageusement un tel microgranulé pour lutter contre les champignons du sol, ou contre les champignons pathogènes des tiges et des parties aériennes traités en application au sol ou dans l'eau, en particulier dans les rizières.

Les composés de formule (I) peuvent encore être utilisés sous forme de poudres pour poudrage ; on peut aussi utiliser une composition comprenant 50 g de matière active et 950 g de talc ; on peut aussi utiliser une composition comprenant 20 g de matière active, 10 g de silice finement divisée et 970 g de talc ; on mélange et broie ces constituants et on applique le mélange par poudrage.

Les exemples ci-dessous illustrent l'invention :

Exemple 1 : Préparation de 2-(2,4-dichloro 1-phényl) 2-méthyl (1,2,4-triazole 1-yl) 2,5-dihydrofuranne

Dans un ballon de 50 ml sous atmosphère inerte, on introduit une solution de 2-chlorométhyl 2-(2,4-dichloro 1-phényl)2,5-dihydrofuranne (1,001 g ; 3,80 mmoles) dans la N-méthyl pyrrolidone (N.M.P.) (1,00 g). On ajoute alors le triazole -1,2,4 (314, 8 mg ; 4,56 mmoles) et du carbonate de potassium (630,2 mg ; 4,56 mmoles). Le milieu réactionnel est porté à 170° C pendant 14 heures puis est refroidi au voisinage de 20° C. On ajoute alors 10 ml de toluène puis de l'eau saturée de chlorure d'ammonium. La phase toluénique est recueillie, la phase aqueuse est extraite avec encore 2 fois 10 ml de toluène. Les extraits organiques réunis sont séchés sur sulfate de sodium. Après filtration et concentration sous pression réduite, on obtient un résidu semi cristallin qui est purifié.

Poids obtenu : 673 mg (2,28 mmoles) ; PF (Kofler) : 107° C.

Rendement 60 % par rapport au dihydrofuranne-2,5 de départ.

Préparation du 2-chlorométhyl 2-(2,4-dichloro 1-phényl)2,5-dihydrofuranne

Dans un ballon de 100 ml sous atmosphère inerte, on introduit 10 g (35,5 mmoles) de 1-chloro 2-(2,4-dichloro 1-phényl) 3-pentène 2,5 diol (isomère cis) dans 60 ml de toluène. On ajoute 0,5 g d'acide paratoluène sulfonique et on porte au reflux. La réaction terminée on lave et sépare. On obtient une résidu huileux brun 9,36 g (35,5 mmoles).Rendement 100 % par rapport au produit de départ.

Préparation du 1-chloro 2-(2,4-dichloro 1-phényl) 3-pentène 2,5 -diol (isomère cis)

Méthode A :

Dans un ballon de 500 ml sous atmosphère inerte on introduit 24 g de 1-chloro 2-(2,4-dichloro 1-phényl) 5-[(1-éthoxyéthoxy)] 3- pentyne 2-ol en solution dans le toluène (150 ml), 0,613 g de palladium à 5% sur charbon. On alimente en hydrogène sous pression atmosphèrique à 25° C. Au bout de 2 heurs, on filtre puis le solvant est éliminé sous pression réduite.

Le résidu huileux est repris au méthanol (200 ml) et on ajoute de l'acide chlorhydrique 0,5 N (50 ml). Le méthanol est alors éliminé sous pression réduite et on obtient 19,36 g d'un résidu huileux orange.

L'addition de 10 ml d'acétate d'éthyle puis de 35ml de pentane permet la précipitation de 5,35 g (19,3mmoles) de diol souhaité.

Préparation du 1-chloro 2-(2,4-dichloro 1-phényl) 5-[(1-éthoxyéthoxy)] 3-pentyne 2-ol.

Dans un ballon de 500 ml sous atmosphère inerte et contenant 13,37 g de magnésium et 30 ml de tétrahydrofuranne on coule en 2h30 54,5 g (0,5 moles) de bromoéthane en solution dans 225 ml de THF à T = 30° C. On coule la solution obtenue goutte à goutte en 1 heure à température ambiante sur une solution d'éther de 2-éthoxyéthyle et de propargyle (64,09 g ; 0,5 mole) dans le THF (40 ml). Dans la solution précédente on coule à 0° C en 2 heures une solution de 2,4,2'-trichloroacétophénone (89,4 g ; 0,4 moles) dans 100 ml de THF. On maintient 6 h à 20° C. On refroidit à 0° C et on ajoute en 15 mn à 5° C de l'acide acétique (28,6 ml ; 0,5 mole) puis 150 ml d'eau puis 100 ml d'éther éthylique. La phase organique est lavée avec 2 fois 50 ml d'eau et une fois avec 50 ml de saumure puis les solvants sont éliminés sous pression réduite. On obtient 136,6 g d'une huile jaune visqueuse.

Rendement : 97,2 % par rapport au produit de départ.

Méthode B :

Dans un ballon de 1 l sous atmosphère inerte et contenant du magnésium (36,5 g ; 1,5 moles) et THF (216 g) et du toluène 75 ml en solution, on coule à 30° C en 1h15, 163,45 g de bromoéthane dans 250 ml de toluène. On laisse reposer 15 mn à 24° C. On coule goutte à goutte une solution d'alcol propargylique (42,15 g ; 0,75 mole) dans le toluène (50 ml) en 1h30. On ajoute goutte à goutte à 44° C une solution de 2,4,2'-trichloroacétophénone (111,7 g ; 0,5 mole) dans le toluène (100 ml). On laisse reposer à température ambiante. Le milieu est alors refroidi à 0° C et on ajoute goutte à goutte 90 g (1,5 mole) d'acide acétique. On évapore, ajoute du toluène (250 ml) et lave à l'acide sulfurique dilué puis à l'eau. La phase organique est alors concentrée, refroidie, précipitée, filtrée, séchée. On obtient le 1-chloro 2-(2,4-dichloro 1-phényl) 3-pentyne 2,5 diol. P.F = 90° C. L'hydrogénation est effectuée de la même manière que pour la méthode A mais à 50° C. On obtient le diol souhaité.

Préparation du composé de l'exemple 1 selon une autre méthode.

Etape a) Préparation du 1-chloro 2-(2,4-dichlorophényl) 4-pentene 2-ol:

Un dérivé organomagnésien est préparé par addition d'une solution de bromure d'allyle (110 ml) dans l'éther éthylique (700 ml) et le tétrahydrofuranne (200 ml) avec du magnésium (110 g) entre 15 et 20° C en trois heures. On chauffe à reflux 30 mn, décante, évapore la phase organique et lave à l'éther le résidu.

On additionne à -30° C une solution de trichloro-alpha, 2, 4 acétophénone (175 g) dans le tétrahydrofuranne (250 g), neutralise à l'acide acétique. On lave à l'eau, séche sur sulfate de sodium, concentre puis distille sous vide. Une huile incolore est obtenue (205 g). Point d'ébullition (3 $10^{-2}$ mmHg) = 140-142° C.

Etape b) Préparation du 1-[2-(2,4-dichlorophényl)2-hydroxy 4-pentènyl] 1 H 1,2,4-triazole

Un mélange de produit obtenu à l'étape a) (106 g), de triazole (55 g) et de carbonate de potassium (160 g) est chauffé pendant quatre heures à 120° dans le diméthylformamide (600 ml). Les insolubles sont filtrés, lavés au diméthylformamide et le mélange réactionnel est concentré sous vide. Le résidu, dissous dans le chlorure de méthylène, est lavé à l'eau puis concentré. Le produit est obtenu par cristallisation dans l'acétate d'éthyle après dilution à l'heptane. Un solide rose pâle (97 g) est isolé fondant à 101°.

Etape c) Préparation du 1-[4-bromo 2-(2,4-dichlorophényl) tétrahydro 2-furanylméthyl] 1H 1,2,4-triazole

Le composé obtenu à l'étape b (35 g) dans le chloroforme (200 ml) est traité à 0° par du brome. Après décoloration, le solvant est évaporé et le résidu redissous dans le méthanol. On ajoute ensuite une solution aqueuse de potasse jusqu'à obtention d'un pH basique. Après évaporation du milieu sous vide, le résidu est extrait à l'acétate d'éthyle, lavé à l'eau et concentré. L'huile obtenue (40 g) est constituée d'un mélange de deux diastéréoisomères en proportions sensiblement égales. Par chromatographie sur silice on isole successivement l'isomère le moins polaire no1a : cristaux blancs fondant à 83°, puis l'isomère le plus polaire no1b : cristaux blancs fondant à 94°. Après recristallisation on obtient 1a fondant à 96° et 1b fondant à 104°.

Etape d) Préparation de 2-(2,4-dichloro 1-phényl) 2-méthyl (1-triazolyl) 2,5-dihydrofuranne

Dans un ballon de 250 ml on introduit l'isomère a (30 g ; 795 mmoles) du méthanol (200 ml) de la potasse en pastille à 85 % (10,4 g ; 159 mmoles). Le milieu réactionnel est porté à reflux pendant 1 heure, refroidit, filtré puis concentré. Le résidu est redissout dans du chloroforme (200 ml), filtré et évaporé. Le résidu semi-solide est recristallisé dans l'acétate d'éthyle avec 20 % d'heptane.

Poids obtenu : 19, 4 g    PF : 107°

Exemple no2 : Préparation de 2-(2,4-dichloro 1-phényl) 2-(méthyl 1,2,4-triazole 1-yl) 4-n-propyl 2,5-dihydrofuranne :

(composé no2)

Dans un ballon de 1 l sous azote, on place un mélange d'alcool propargylique (7,8 ml - 0,132 mole), d'iodure cuivreux (2,6 g - 0,014 mole) et de tétrahydrofuranne sec que l'on refroidit à - 10°. On additionne goutte à goutte à cette température un organomagnésien préparé à partir de bromure de propyle (34,4 g - 0,28 mole) et de magnésium (7,2 g - 0,30 mole) dans le tétrahydrofuranne (120 ml). Après 15 heures à température ambiante on additionne goutte à goutte une solution de 2,4,2'-trichloro-acétophénone (23,6 g - 0,106 mole) dans le tétrahydrofuranne (140 ml). Après 2 heures le milieu est neutralisé à l'acide acétique glacial versé dans l'eau et extrait à l'éther, séché puis évaporé. On obtient une huile orangée (17 g) constituée essentiellement de 2-(2,4-dichlorophényl) 1-chloro 4-propyl 3-cis-pentène-2,5 diol qui est acétylé en présence d'anhydride acétique (5,6 ml) dans la pyridine (30 ml). Après une nuit à température ambiante, la pyridine est coévaporée avec du toluène et le résidu est chauffé à 130° pendant 3 heures en présence de carbonate de potassium (25 g) et de triazole (7 g) dans le diméthylformamide (250 ml). Le milieu est filtré, concentré, dilué à l'eau et extrait au dichlorométhane. Après évaporation du solvant, le résidu est chromatographié sur la silice (éluant acétate d'éthyle - heptane 1 : 1) et fournit le composé 2 sous forme de cristaux faiblement colorés en jaune (4,2 g) fondant à 85°.

Exemple 3 : Préparation de 2-(4-chlorophényl) 2-(méthyl-1,2,4-triazole 1-yl) 4-éthyl 2,5 dihydrofuranne

(composé no3)

On procède de la manière identique à l'exemple ci-dessus en utilisant le bromure d'éthyle (27,2 g - 0,25 mole) et la 4,2'-dichloroacétophénone (20,8 g - 0,11 mole). Après condensation avec le triazole, la cyclisation n'est pas effectuée et on obtient essentiellement le monoacétate du 2-(4-dichlorophényl) 1-(1,2,4-triazole 1-yl) 4-éthyl 3-cis pentène 2,5-diol brut (20 g d'une huile noire). Celui-ci est alors saponifié par la potasse (5,8 g) dans le méthanol (250 ml) à reflux. Après évaporation, dilution à l'eau, extraction au dichlorométhane et évaporation sous vide, une huile noire est obtenue (17,5 g). Celle-ci en solution dans le dichlorométhane sec (200 ml) est traitée par du chlorure de méthane sulfonyle (4,9 ml) puis à -30° par de la triéthylamine goutte à goutte. Après 2 heures, le milieu est lavé à l'eau, concentré et le résidu, redissous dans le méthanol (200 ml), est traité par de la potasse (3,8 g). Après extraction à l'acétate d'éthyle et lavage à l'eau, le résidu brut est chromatographié, (éluant : acétate d'éthyle - heptane 1 : 1). Le produit attendu est dientifié aisément par sa révélation intense à l'iode en chromatographie couche mince. On obtient la produit 3 sous forme d'une huile brune partiellement cristallisée. Selon les méthodes ci-dessus les composés de formule XV ont été préparés.

| | $X_1$ | $Y_1$ | $R_3$ | $R_4$ | F |
|---|---|---|---|---|---|
| 1 | Cl | Cl | H | H | 107° |
| 2 | Cl | Cl | n-propyl | H | 85° |
| 3 | Cl | H | éthyl | H | huile |
| 4 | Cl | H | H | H | 124° |
| 5 | Cl | Cl | éthyl | H | 86,5° |
| 6 | Cl | H | n-propyl | H | |
| 7 | Cl | Cl | 4-F phényl | H | |
| 8 | Cl | Cl | phényl | H | |
| 9 | Cl | Cl | allyl | H | |
| 10 | Cl | H | allyl | H | |
| 11 | Cl | Cl | 3-F propyl | H | |
| 12 | Cl | H | 3-F propyl | H | |
| 13 | Cl | Cl | cyclopentyl | H | |
| 14 | Cl | H | cyclopentyl | H | |
| 15 | Cl | Cl | allyl | $CH_3$ | |

1 2-(2,4-dichloro 1-phényl) 2-méthyl (1,2,4-triazole 1-yl) 2,5-dihydrofuranne
2 2-(2,4-dichloro 1-phényl) 4-n propyl 2-méthyl (1,2,4-triazole 1-yl) 2,5-dihydrofuranne
3 2-(4-chloro 1-phényl) 4-éthyl 2-méthyl (1,2,4-triazole 1-yl) 2,5-dihydrofuranne
4 2-(4-chloro 1-phényl) 2-méthyl (1,2,4-triazole 1-yl) 2,5-dihydrofuranne
5 2-(2,4-dichloro 1-phényl) 4-éthyl 2-méthyl (1,2,4-triazole 1-yl) 2,5-dihydrofuranne
6 2-(4-chloro 1-phényl) 4-n propyl 2-méthyl (1,2,4-triazole 1-yl) 2,5-dihydrofuranne
7 2-(2,4-dichloro 1-phényl) 4-F phényl 2-méthyl (1,2,4-triazole 1-yl) 2,5-dihydrofuranne
8 2-(2,4-dichloro 1-phényl) 4-phényl 2-méthyl (1,2,4-triazole 1-yl) 2,5-dihydrofuranne
9 2-(2,4-dichloro 1-phényl) 4-allyl 2-méthyl (1,2,4-triazole 1-yl) 2,5-dihydrofuranne
10 2-(4-chloro 1-phényl) 4-allyl 2-méthyl (1,2,4-triazole 1-yl) 2,5-dihydrofuranne
11 2-(2,4-dichloro 1-phényl) 3-F n propyl 2-méthyl (1,2,4-triazole 1 yl) 2,5-dihydrofuranne
12 2-(4-chloro 1-phényl) 3-F n propyl 2-méthyl (1,2,4-triazole 1-yl) 2,5-dihydrofuranne
13 2-(2,4-dichloro 1-phényl) 4-cyclopentyl 2-méthyl (1,2,4-triazole 1-yl) 2,5-dihydrofuranne
14 2-(4-chloro 1-phényl) 4-cyclopentyl 2-méthyl (1,2,4-triazole 1-yl) 2,5-dihydrofuranne
15 2-(2,4-dichloro 1-phényl) 4-allyl 3-méthyl 2-méthyl (1,2,4-triazole 1-yl) 2,5-dihydrofuranne

Exemple 16 - Test in vivo sur Erysiphe graminis sur orge (oïdium de l'orge)

De l'orge, en godets, semée dans de la terre franche, est traitée au stade 10 cm de hauteur par pulvérisation d'une émulsion aqueuse (appelée bouillie) de la matière active à tester ayant la composition suivante :
- composé de l'exemple 1 ou 2 : 60 mg
- Tween 80 (agent tensio actif) constitué d'un oléate de dérivé polyéxyéthylèné du sorbitan) dilué à 10 % dans l'eau : 0,3 ml
- on complète à 60 ml d'eau.

Cette émulsion aqueuse est ensuite diluée par de l'eau pour obtenir la concentration désirée. L'essai est répété deux fois. Au bout de 24 heures, on saupoudre les plants d'orge avec des spores d'Erysiphe graminis, le saupoudrage étant effectué à l'aide de plants malades.

La lecture se fait 8 à 14 jours après la contamination.

Dans ces conditions, on observe à la dose de 0,33g/l, une protection totale pour les composés 1, 2, 3, 4, 5.

Exemple 17 - Test in vivo sur "Puccina recondita" responsable de la rouille du blé

Du blé, en godets, semé dans de la terre franche, est traité au stade 10 cm de hauteur par pulvérisation avec des émulsions aqueuses (appelées bouillies) de même composition que celle décrite à l'exemple 16.

Au bout de 24 heures, une suspension aqueuse de spores (50000 sp/$cm^3$) est pulvérisée sur le blé ; cette suspension a été obtenue à partir de plants contaminés. On place ensuite le blé pendant 48 heures en cellule d'incubation à environ 18°C et à 100 % d'humidité relative.

Au bout de ces 2 jours, l'humidité relative est ramenée à 60 %. Le contrôle de l'état des plants se fait entre le 11ème et le 15ème jour après la contamination par comparaison avec le témoin non traité.

A la dose de 0,33g/l, protection totale pour les composés 1, 2, 5.

Exemple 18 - Test in vivo sur "piricularia oryzae" responsable de la Piriculariose du riz (Rice Blast)

Du riz, en godets, semé dans un mélange 50/50 de tourbe enrichie et de pouzzolane, est traité au stade 10 cm de hauteur par pulvérisation d'une émulsion aqueuse (appelée bouillie) ci-dessus définie de concentration indiquée ci-après. L'essai est répété deux fois. Au bout de 48 heures, on traite par application sur les feuilles, par une suspension de spores obtenues en culture pure.

La lecture se fait 8 jours après la contamination. Dans ces conditions, on observe à 0,33g/l, une protection totale pour les composés 1, 2, 3, 5.

Exemple 19-Test in vivo sur Botrytis Cinerea de la tomate.

On prépare par broyage fin une suspension aqueuse de la matière active à tester ayant la composition suivante :

- matière active à tester........................ 60 mg
- Tween 80 (agent tensioactif constitué d'un monolaurate de dérivé polyoxyéthylèné du sorbitan.............. 0,6 ml
- eau........................................ 60 ml

Cette suspension aqueuse est ensuite diluée par de l'eau pour obtenir la concentration désirée.

Des tomates cultivées en serre (variété Marmande) agées de 60 à 75 jours sont traitées par pulvérisation avec des suspensions aqueuses de la composition décrite à concentration de matière active de 1 g/l (1000 ppm). L'essai est répété deux fois avec chaque concentration.

Après 24 heures, les feuilles sont coupées et mises dans 2 boîtes de Piétri (diamètre 11 cm) dont le fond a été préalablement garni d'une disque de papier filtre humide (5 folioles par boîte).

L'inoculum est ensuite apporté à l'aide d'une seringue par dépôt de gouttes (3 gouttes par foliole) d'une suspension de spores. Cette suspension de spores de Botrytis cinerea a été obtenue à partir d'une culture de 15 jours, mise ensuite en suspension dans une solution nutritive (80.000 unités/$cm^3$). Le contrôle est fait environ 4 à 6 jours après le contamination par comparaison avec un témoin non traité. On évalue ainsi le pourcentage de protection par rapport au témoin non traité.

Dans ces conditions, on observe que, à la concentration de 0,33g/l, une protection totale pour les composés suivants : 2, 5.

Exemple 20 - Test in vitro sur champignons des semences et champignons du sol

On étudie l'action des composés selon l'invention sur les champignons suivants responsables des maladies des céréales et autres végétaux :

11) Pyrenophorae avenae
6) Septoria nodorum
12) Helminthosporium teres
9) Fusarium roseum
8) Fusarium nivale
7) Fusarium culmorum
13) Rhizoctonia cerealis
14) Septoria tritici
1) Botrytis cinerea sensible au carbendazime et aux imides cycliques
2) Botrytis cinerea résistant au carbendazime et aux imides cycliques
5) Pseudocercosporella herpotrichoïdes
3) Fusarium oxysporum F.sp melonis
4) Rhizoctonia solani
10) Helminthosporium gramineum

Les chiffres figurant avant les noms seront utilisés pour identifier les champignons dans le tableau

ci-dessous.

Pour chaque essai, on opère de la manière suivante : un milieu nutritif constitué de pomme de terre, de glucose et de gelose (milieu PDA) est introduit en surfusion dans une série de boîtes de Pétri (20 ml par boîte) après stérilisation à l'autoclave à 120° C.

Au cours du remplissage des boîtes, on injecte, dans le milieu en surfusion, une solution acétonique de la matière active, pour obtenir la concentration finale désirée.

On prend comme témoin des boites de Pétri analogues aux précédentes, dans lesquelles on coule des quantités similaires d'un milieu nutritif ne contenant pas de matière active.

Après 24 ou 48 h chaque boîte est ensemencée par dépôt d'un fragment de mycelium provenant d'une culture précédente du même champignon.

Les boîtes sont conservées pendant 2 à 10 jours (selon le champignon testé) à 22° C et on compare alors la croissance du champignon dans les boîtes contenant la matière active à tester, à celle du même champignon dans la boîte utilisée comme témoin.

On détermine ainsi pour chaque composé testé le taux d'inhibition du champignon considéré pour une dose de 30 ppm.

| | 11 | 6 | 12 | 9 | 8 | 7 | 13 | 14 | 1 | 2 | 5 | 3 | 4 | 10 |
|---|---|---|---|---|---|---|---|---|---|---|---|---|---|---|
| 1 - | 95 | 95 | 100 | 90 | 90 | 100 | 95 | 100 | 100 | 100 | 100 | 80 | 80 | 100 |
| 2 - | 95 | 90 | 100 | 80 | 100 | 100 | 95 | - | 95 | 95 | 90 | 80 | 90 | 95 |
| 3 - | 90 | 95 | 90 | 50 | 50 | 90 | 90 | - | 80 | 90 | 100 | 50 | 50 | 90 |
| 4 - | 100 | 90 | 100 | 90 | 90 | 100 | 100 | - | 100 | 100 | 100 | 100 | 80 | 100 |
| 5 - | 90 | 100 | 90 | 50 | 90 | 95 | 80 | - | 90 | 100 | 100 | 80 | 80 | 90 |

# FORMULE DES COMPOSES

$$(X)_n Ph-(CH_2)_m-C(OH)(CH_2Hal)-C \equiv C-CR_1R_2OPr \qquad (IV)$$

$$(X)_n Ph-(CH_2)_m-C(OH)(CH_2Hal)-CR_4=CR_3-CR_1R_2-OZ \qquad (III)$$

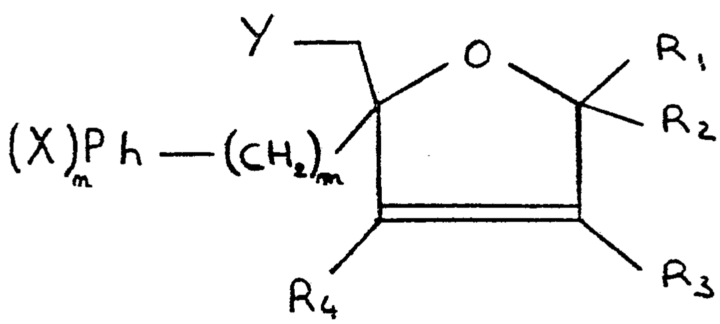

(II)

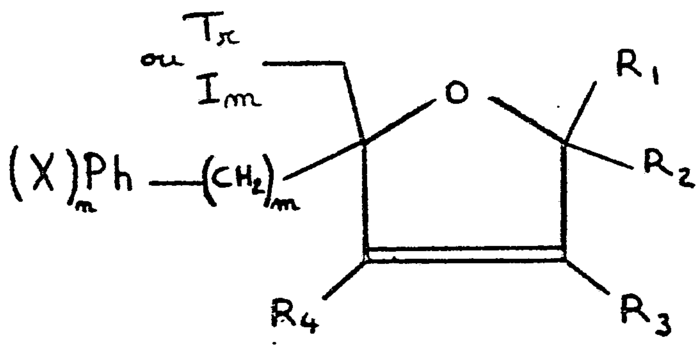

(I)

Tr = Triazolyl (W = -CH=) Im = Imidazolyl (W = -N=)

Ph = phényle

## FORMULE DES COMPOSES

$(X)_nPh-(CH_2)_m-C(OH)(CH_2Hal)-CHR_4-CR_3=CR_1R_2$ (V)

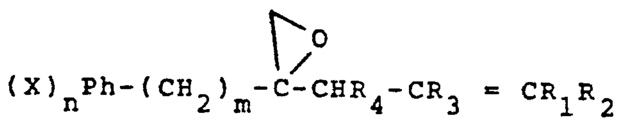

(VII)

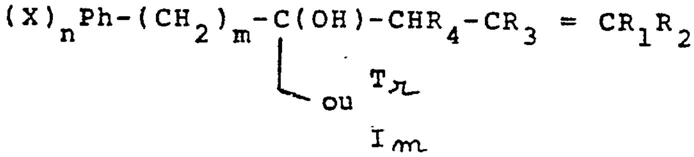

(VI)

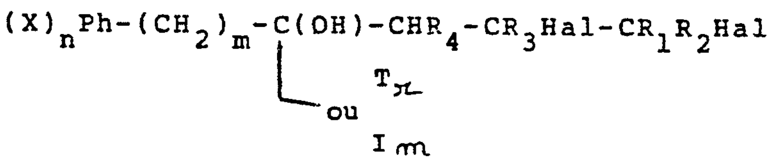

(VIII)

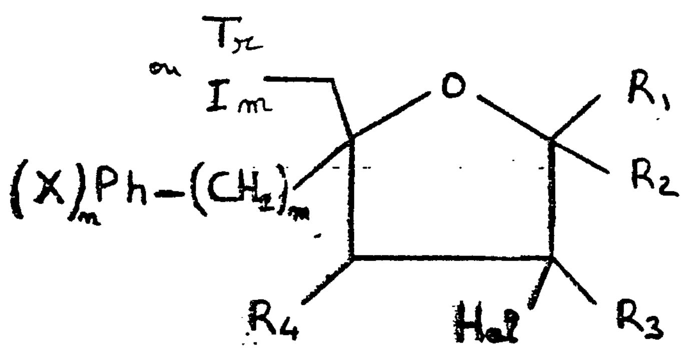

(IX)

## FORMULE DES COMPOSES

$(X)_nPh-(CH_2)_m-C(OH)(CH_2Hal)-CHR_4-CR_3Hal-CR_1R_2Hal$ (X)

$(X)_nPh-(CH_2)_m-C(OH)-C \equiv C-CR_1R_2OPr$ (XII)

Tr
ou
Im

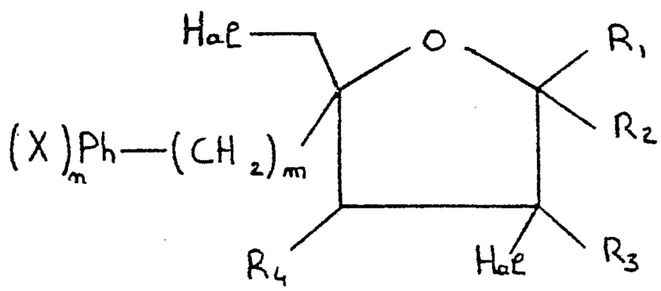

(XI)

$(X)_nPh-(CH_2)_m-C(OH)-CR_4 = CR_3-CR_1R_2OZ$ (XIII)

Tr
ou
Im

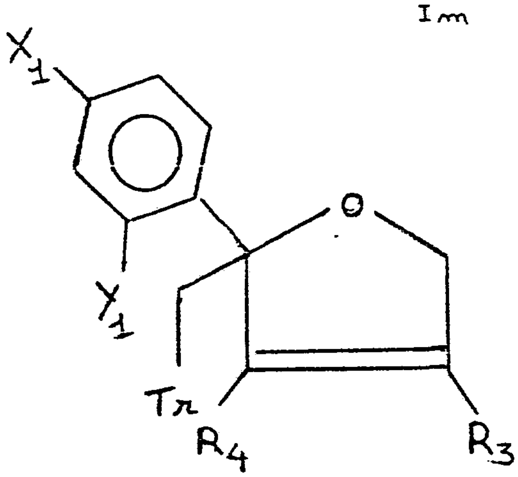

XV

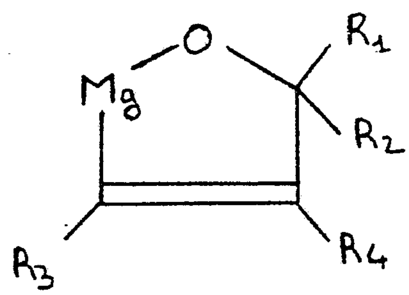

XIV

## Revendications

1 Composés de formule I indiquée dans la description dans laquelle
- $R_1$, $R_2$, $R_3$, $R_4$, identiques ou différents, sont l'atome d'hydrogène, ou un radical alkyle inférieur, cycloalkyle inférieur, aryle (notamment phényle), éventuellement substitué par un ou plusieurs atomes ou radicaux comme les atomes d'halogènes, les radicaux alkoxy inférieurs, aryloxy, aryle, alkyle inférieur, halogénoalkyle inférieur, halogénoalcoxy inférieur, hydroxy,
- X est un atome d'halogène, de préférence le fluor, le brome ou le chlore, ou un groupe alkyle ou alkoxy ayant de 1 à 12 atomes de carbone, de préférence de 1 à 4 atomes de carbone, et étant éventuellement mono ou polyhalogéné (groupement $CF_3$ notamment), ou un groupe cyano,
- n est un nombre entier, positif ou nul, inférieur à 6, étant entendu que lorsque n est supérieur à 1, les substituants X peuvent être soit identiques, soit différents,
- m = 0 ou 1, de préférence 0,
- Ph est le noyau phényle, éventuellement substitué (n= 1 à 5),
- - Tr représente un groupe 1,2,4-triazole 1-yl et Im représente un groupe 1,3-imidazolyl 1-yl, de préférence les composés comportent un groupe 1,2,4-triazole 1-yl et leurs formes salifiées.

2) Composés selon la revendication 1 caractérisés en ce que X correspond à l'atome d'halogène, de préférence le chlore et n = 1, 2 ou 3.

3) Composés selon la revendication 2 caractérisés en ce que n=2, de préférence X est en ortho et para,

4) Composés selon la revendication 1 caractérisés en ce que $R_1$, $R_2$ correspondent à l'atome d'hydrogène, et avantageusement
$R_3$, $R_4$ identiques ou différents correspondent à un radical alkyl inférieur,
ou
$R_3$ correspond à un radical alkyle inférieur et $R_4$ est l'atome d'hydrogène
ou
$R_4$ correspond à un radical alkyle inférieur, et $R_3$ est l'atome d'hydrogène.

5) Procédé de préparation des composés selon la revendication 1 caractérisé en ce qu'il consiste dans les étapes suivantes :
- hydrogénation, dans le cas où $R_3$ et $R_4$ correspondent à l'atome d'hydrogène, d'un composé de formule IV, dans laquelle X, n, m, $R_1$, $R_2$ ont la même signification que dans la revendication I, Pr est un groupe protecteur, Hal correspond à un atome d'halogène ou, dans le cas où $R_3$ et/ou $R_4$ sont différents de l'atome d'hydrogène addition d'un organomagnésien de formule $R_4MgX$ sur le composé de formule IV puis éventuellement addition d'un halogénure d'alkyle de formule $R_3X$,
- cyclisation du composé de formule III dans laquelle $R_1$, $R_2$, $R_3$, $R_4$, X, n, m ont la même signification que dans la revendication I, Hal a la même signification que dans le composé de formule IV, Z est un atome d'hydrogène ou OZ est un groupe partant
- greffage d'un noyau imidazole ou triazole sur le composé de formule II dans laquelle $R_1$, $R_2$, $R_3$, $R_4$, X, m, n ont la même signification que dans la revendication 1 et Y correspond à un atome ou groupement susceptible d'être éliminé au moyen d'une substitution nucléophile, éventuellement après transformation intermédiaire appropriée.

6) Procédé selon la revendication 5, caractérisée en ce que l'hydrogénation est effectué au moyen d'un catalyseur choisi parmi le groupe suivant : palladium, ruthénium, nickel de Rancy, platine, rhodium, déposé sur un support inerte.

7) Procédé selon la revendication 5, caractérisé en ce que l'addition de $R_4MgX$ est effectué en présence d'iodure de cuivre.

8) Procédé selon la revendication 5, caractérisé en ce que lorsque Y est un atome d'halogène, on cyclise le composé de formule III soit en milieu acide si Z est un atome d'hydrogène, soit en milieu basique si Z est un groupe partant.

9) Procédé selon la revendication 8, caractérisé en ce que la réaction de cyclisation est effectuée en présence de 0,1 à 2 équivalent molaire d'acide par mole de composé de formule III.

10)Procédé selon la revendication 8, caractérisé en ce que la réaction de cyclisation est effectuée en présence de 0,01 à 2 équivalent molaire de base par mole de composé de formule III.

11)Procédé selon la revendication 8, caractérisé en ce que la température de la réaction est comprise entre 10° C et 100° C ou s'il y a un solvant entre 10° C et la température d'ébullition du solvant considéré.

12)Procédé selon la revendication 5 caractérisé en ce que lorsque Y est un groupe hydroxy ou un groupe partant OZ, OZ étant de préférence le tosylate, le mésylate, le triflate ou le groupement de formule [$PH_3P^+$-$O^-$] il consiste à traiter par une base le composé de formule III dans laquelle Z est un atome d'hydrogène, et $R_1$, $R_2$, $R_3$, $R_4$, X, m, n, ont la même signification que dans la revendication 1, puis à transformer éventuellement le groupe Y = OH en groupe partant OZ.

13)Procédé selon la revendication 12 caractérisé en ce que 1'on utilise 0,01 à 2 équivalents molaire de base par mole de composé de formule III.

14)Procédé selon la revendication 5, caractérisé en ce que l'étape de greffage est effectuée au moyen d'un dérivé alcalin, de phosphonium, ou d'ammonium d'un imidazole ou triazole formé ou non in situ, et en ce que le rapport molaire dudit dérivé par rapport au composé de formule I est de préférence compris entre 1,05 et 1,5.

15)Procédé selon l'une des revendications 5 ou 14, caractérisé en ce que la réaction de l'étape de greffage est effectuée en présence d'un solvant et en ce que la quantité de composé de formule III par rapport au poids total de solution est comprise entre 1 et 70 % en poids.

16)Procédé selon l'une des revendications 5, 14 ou 15, caractérisé en ce que la témpérature de la réaction de l'étape de greffage est voisine de la température d'ébullition du solvant.

17)Procédé de préparation des composés selon la revendication 1 caractérisé en ce qu'il consiste dans les étapes suivantes :

- addition d'une halogénoacétophénone de formule : $(Xn)PH(CH_2)_mCO\ CH_2Hal$ sur un organométallique de formule $R_2R_1C = CR_3\text{-}CHR_4M$ dans lesquelles X, m, n, Hal, $R_1$ à $R_4$ ont la même signification que dans les revendications 1 et 5 et M correspond à un métal alcalin, à un magnésien ou à un zincique,
- greffage d'un noyau triazole ou imidazole sur le composé de formule V dans laquelle X, m, n, Hal, $R_1$ à $R_4$ ont la même signification que dans les revendications 1 et 5, dans les mêmes conditions opératoires que celles revendiquées aux revendications 14 à 16, de manière à obtenir le composé de formule VI.
- addition sur le composé de formule VI d'une molécule d'halogène ou d'halogène mixte de manière à conduire au composé de formule VIII,
- cyclisation du composé de formule VIII de manière à obtenir le composé de formule IX
- reaction du composé de formule IX avec une base de manière à obtenir le composé de formule I.

18)Procédé de préparation des composés selon la revendication 1 caractérisé en ce qu'il consiste à greffer le noyau triazole ou imidazole sur les composés de formules IV ou III dans lesquelles X, n, m, Hal, $R_1$ à $R_4$, Pr, Z ont la même signification que dans la revendication 5, dans les mêmes conditions que celles revendiquées aux revendications 14 à 16, de manière à obtenir les composés de formule XII et XIII respectivement puis à hydrogéner dans le cas du composé XII, de préférence dans les mêmes conditions que celles indiquées dans la revendication 6, puis à cycliser, de préférence dans les mêmes conditions que celles indiquées aux revendications 8 à 13.

19)Utilisation des composés selon l'une des revendications 1 à 4 à titre de fongicide.

20)Compositions fongicides, caractérisées en ce qu'elles contiennent comme matière active un composé selon l'une des revendications 1 à 4, cette matière active étant en association avec au moins un support inerte, acceptable en agriculture.

21)Compositions selon la revendication 20, caractérisées en ce qu'elles contiennent 0,5 à 95 % de matière active.

22)Procédé pour lutter contre les maladies fongiques des cultures, caractérisé en ce qu'on applique une dose efficace d'une matière active selon l'une des revendications 1 à 4.

23)Procédé pour lutter contre les maladies fongiques des cultures selon la revendication 22, caractérisé en ce que la matière active est appliquée à raison de 0,005 à 5 kg/ha, de préférence de 0,01 à 0.5 kg/ha.

24)Composé de formules II, III, IV, dans lesquels X, n, m, $R_1$ à $R_4$, Y ont la même signification que dans les revendications 1 et 5.